# EUROPEAN PATENT APPLICATION

(11) **EP 1 526 135 A1**
(43) Date of publication of application: **27.04.2005**
(21) Application number: 03292660.2
(22) Date of filing: 24.10.2003
(51) Int. Cl.: C07D 471/16, A61K 31/55

(54) **Azabenzodiazepines as pde4 inhibitors**

(71) Applicant: Warner-Lambert Company LLC, Morris Plains, New Jersey 07950 (US)
(72) Inventor: Jacobelli, Henry, Sandwich,Kent CT13 9NJ (GB); Devillers, Ingrid, Sandwich,Kent CT13 9NJ (GB); Pevet, Isabelle, Sandwich,Kent CT13 9NJ (GB); Gaudilliere, Bernard, Sandwich,Kent CT13 9NJ (GB)
(74) Representative: Laurent, Claire

(57) **Abstract**

Compounds of formula (I): 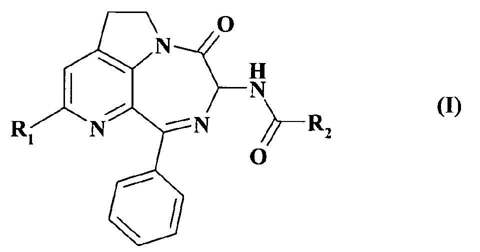 characterized in that:
- R₁ represents a group selected from hydrogen atom, methyl, methoxy, hydroxy, amino, dimethylamino, acetamido, pyrrolidin-1-yl, and hydroxymethyl;
- R₂ represent a group selected from phenyl, pyridyl, pyrimidyl, quinolyl, isoquinolyl, indolyl, pyrolyl, [1,2,3]-triazolyl, benzo[c]isoxazolyl, thienyl, pyrazolyl, isothiazolyl, imidazolyl, benzofuranyl, pyrazolo[5,1-c][1,2,4]triazyl each of these groups being optionally substituted from 1 to 3 groups, identical or different independently of each other, selected from halogen, trifluoromethyl, (C₁-C₄)alkyl, (C₁-C₄)alkoxy, hydroxy, amino, acetamido, tert-butyloxycarbonylamino, cycloalkylcarbonylamino, sulfonamide, nitro, acetylmethoxy, cyclopentyloxy;
optionally, their optical isomers, and addition salts thereof with a pharmaceutically acceptable acid or base,
and their use as active ingredient in pharmaceutical composition useful for treating diseases involving therapy by inhibition of PDE4.

## Description

### FIELD OF THE INVENTION

The present invention relates to azabenzodiazepine compounds, to pharmaceutical compositions containing them, to their use as medicinal products and to process for preparing them. These compounds are useful for preparing medicinal products for treating complaints involving a therapy with a phosphodiesterase-4 (PDE4) inhibitor. These medicinal products are useful in particular for treating certain inflammatory conditions and allergic conditions and are more particularly useful in the treatment of various respiratory diseases such as asthma, emphysema and chronic bronchitis.

### TECHNOLOGICAL BACKGROUND OF THE INVENTION

Cyclic adenosine 3',5'-monophosphate (cAMP) is a ubiquitous intracellular second messenger, which is intermediate between a first messenger (hormone, neurotransmitter or autacoid) and the cellular functional responses: the first messenger stimulates the enzyme responsible for the synthesis of cAMP; depending on the cells concerned, the cAMP then intervenes in a great number of functions: metabolic, contractile or secretory.

The effects of cAMP end when it is degraded by cyclic nucleotide phosphodiesterases, which are intracellular enzymes that catalyse its hydrolysis into inactive adenosine 5'-monophosphate.

At least 11 major families of cyclic nucleotide phosphodiesterases (PDE) have been distinguished in mammals, numbered from 1 to 11 according to their structure, their kinetic behaviour, their substrate specificity or their sensitivity to effectors (Beavo J.A. *et al.* (1990) Trends Pharmacol. Sci. 11, 150-155. Beavo J.A. *et al.* (1994) Molecular Pharmacol. 46, 399-405). The PDE4 enzymes are specific for cAMP.
Non-specific phosphodiesterase inhibitor compounds are known, which inhibit several families of enzymes. This is the case for certain methyl xanthines such as theophylline. These compounds have a low therapeutic index, in particular on account of their action on types of PDE present in cells other than the target cells. Conversely, certain families of PDE can be selectively inhibited by various pharmacological agents : the hydrolysis of cyclic nucleotides is slowed down and their concentration thus increases in only the cells in which the type of PDE that is sensitive to the inhibitor is found.

A specific advantage is shown for the phosphodiesterases 4 (PDE4), which have been identified in many tissues including the central nervous system, the heart, vascular endothelium, vascular smooth muscle and that of the aerial pathways, myeloid lines and lymphoid lines.

An increase in cAMP in the cells involved in inflammation inhibits their activation: inhibition of the synthesis and release of mediators in mastocytes, monocytes, polymorphonuclear eosinophils and basophils, inhibition of chemotaxis and degranulation of polymorphonuclear neutrophils and eosinophils, inhibition of the proliferation and differentiation of lymphocytes.

Cytokines, in particular TNF and interleukins, produced by various types of leukocytes such as the T lymphocytes, monocytes and polymorphonuclear eosinophils, play an important role in triggering inflammatory manifestations, in particular in response to stimulation by an allergen in the respiratory pathways.

Moreover, cAMP reduces the tonus of the smooth muscle fibres in the aerial pathways.

It might thus be expected that selective PDE4 inhibitors would have therapeutic activity as anti-inflammatory and anti-allergic medicinal products, and in the treatment of various respiratory diseases such as asthma, emphysema and chronic bronchitis.

Extensive research has been conducted for several years into the production and development of powerful PDE4 inhibitors. This is found to be difficult due to the fact that many potential PDE4 inhibitors are not devoid of activity on the phosphodiesterases of other families.

At the present time, the lack of selectivity of PDE4 inhibitors thus represents a major problem, given the extent of the functions regulated by cAMP. There is thus a need for powerful and selective PDE4 inhibitors, i.e. inhibitors which have no action with respect to PDEs belonging to other families and particularly PDEs which regulate cGMP.

### SUMMARY OF THE INVENTION

The present invention relates to compounds of formula (I) : 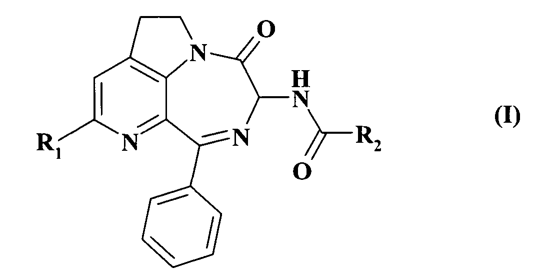
- R₁ represents a group selected from hydrogen atom, methyl, methoxy, hydroxy, amino, dimethylamino, acetamido, pyrrolidin-1-yl, and hydroxymethyl;
- R₂ represent a group selected from phenyl, pyridyl, pyrimidyl, quinolyl, isoquinolyl, indolyl, pyrolyl, [1,2,3]-triazolyl, benzo[c]isoxazolyl, thienyl, pyrazolyl, isothiazolyl, imidazolyl, benzofuranyl, pyrazolo[5,1-c][1,2,4]triazyl each of these groups being optionally substituted from 1 to 3 groups, identical or different independently of each other, selected from halogen, trifluoromethyl, (C₁-C₄)alkyl, (C₁-C₄)alkoxy, hydroxy, amino, acetamido, tert-butyloxycarbonylamino, cycloalkylcarbonylamino, sulfonamide, nitro, acetylmethoxy, cyclopentyloxy ;
and optionally, their optical isomers, and addition salts thereof with a pharmaceutically acceptable acid or base.

According to a first preferred embodiment of the invention:
- R₁ represents a group selected from methyl, methoxy, amino and acetamido;
- R₂ represents a group selected from phenyl, pyridin-3-yl, and pyridin-4-yl, each of these groups being optionally substituted from 1 to 3 groups, identical or different independently of each other selected from halogen, methoxy and amino; and optionally, their optical isomers, and addition salts thereof with a pharmaceutically acceptable acid or base.

According to a second preferred embodiment, the invention relates to compounds of formula (I) wherein R₂ represents a pyridin-4-yl or a pyridin-3-yl group optionally substituted from 1 to 3 groups as defined in claim 1, and optionally, their optical isomers, and addition salts thereof with a pharmaceutically acceptable acid or base.

According to a third preferred embodiment, the invention relates to compounds of formula (I) wherein R₁ represents a group selected from methoxy and amino, and R₂ represents a pyridin-3-yl group, and optionally, their optical isomers, and addition salts thereof with a pharmaceutically acceptable acid or base.

The optical isomers, the N-oxides, as well as the addition salts with a pharmaceutically-acceptable acid or base, of the preferred compounds form an integral part of the invention.

The invention also relates to a pharmaceutical composition comprising as active ingredient an effective amount of a compound of formula (I) alone or together with one or more pharmaceutically-acceptable excipients or carriers.

The invention also relates to a method for treating a living body afflicted with a disease where the inhibition of phosphodiesterase type 4 is involved, the said method comprising the administration of an effective amount of a compound of formula (I) to a patient in need thereof.

Another embodiment of the invention concerns the use of a compound of formula (I), for the preparation of a medicinal product intended for treating a disease involving therapy by inhibition of phosphodiesterase, and more particularly of type-4 phosphodiesterase.

The compounds of the invention can be used in the treatment of complaints including cancer, acquired immunodeficiency syndrome, fibrosis, excessive scarring including excessive dermal scarring such as normal or abnormal dermal scarring following wounding or surgery, osteoarthritis, osteoporosis, multiple sclerosis, anxiety, depression, atopic dermatitis, rheumatoid arthritis, septic shock, immune diseases including disseminated lupus erythematous, psoriasis, graft rejection and allergic rhinitis, as well as diseases involving the production of TNFα and more particularly in the treatment of inflammatory complaints such as asthma, chronic obstructive bronchopneumopathy (COPD), post-ischaemic lesions, pulmonary hypertension, congestive cardiac insufficiency, acute respiratory distress syndrome, and chronic inflammatory diseases of the intestine (IBD) such as Crohn's disease and ulcerative colitis.

### DETAILED DESCRIPTION OF THE INVENTION

The compounds provided by this invention are those defined in formula (I). In formula (I), it is understood that :
- a (C₁-C₄)alkyl group denotes a linear or branched group containing from 1 to 4 carbon atoms ; examples of such groups, without implying any limitation are methyl, ethyl, propyl, isopropyl, tert-butyl,
- a (C₁-C₄)alkoxy group means the alkyl group as mentioned above bound through an oxygen atom ; examples of such compounds without implying any limitation are methoxy, ethoxy, *n*-propyloxy, *tert*-butyloxy,
- a halogen atom means fluoro, chloro, bromo or iodo,
- optical isomers refer to racemates, enantiomers and diastereoisomers.

The invention also relates to the pharmaceutically acceptable salts of the compounds of formula (I). A review of the pharmaceutically acceptable salts will be found in *J. Pharm. Sci.,* 1977, 66, 1-19.
Pharmaceutically acceptable acids mean non-toxic mineral or organic acids. Among those there may be mentioned, without implying any limitation, hydrochloric acid, hydrobromic acid, sulfuric acid, phosphonic acid, nitric acid, citric acid, acetic acid, trifluoroacetic acid, lactic acid, pyruvic acid, malonic acid, succinic acid, glutaric acid, fumaric acid, tartaric acid, maleic acid, ascorbic acid, oxalic acid, methanesulfonic acid, camphoric acid, benzoic acid, toluenesulfonic acid, etc...

Pharmaceutically acceptable bases mean non-toxic mineral or organic bases. Among those, there may be mentioned, without implying any limitation, sodium hydroxide, potassium hydroxide, calcium hydroxide, triethylamine, tert-butylamine, dibenzylethylenediamine, piperidine, pyrrolidine, benzylamine, quaternary ammonium hydroxides etc...

The invention also relates to a process for the preparation of compounds of formula (I), which uses as starting material a compound of formula (II): 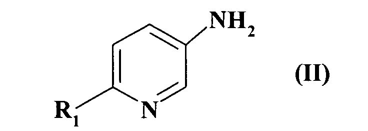 in which R₁ is as defined in the compound of general formula (I),
compound of formula (II) in which the primary amino group is protected in a first step by a usual protective group (G₁) used classically in organic synthesis, then is treated with oxirane or bromoethanol, in presence of a strong base to yield the compound of formula (III): 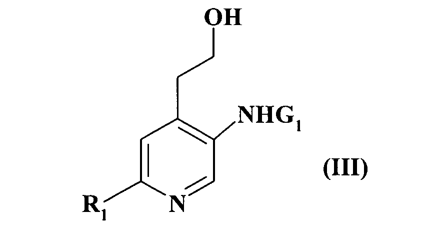 in which R₁ and G₁ are as defined hereinbefore,
compound of formula (III) which is reacted with methanesulfonyl chloride to yield the corresponding mesylate intermediate (in the place of the primary alcohol), which is treated directly in polar condition with LiHMDS to yield to the cyclized compound of formula (IV): 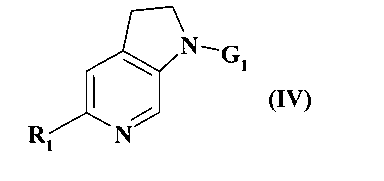 in which R₁ and G₁ are as defined hereinbefore,
compound of formula (IV) which is treated with hydrogen bromide in the presence of hydrogen peroxide to yield to the corresponding 7-bromo-pyrrolo[2,3-c]pyridin of formula (V): 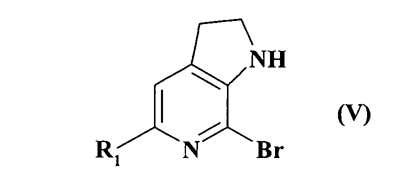 in which R₁ is as defined hereinbefore,
compound of formula (V), which is reacted with zinc cyanide under palladium condition or with cupper cyanide under heating or micro-wave conditions to yield to the corresponding cyanide compound of formula (VI): 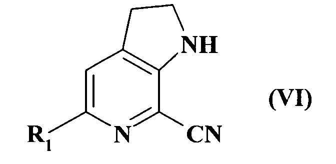 in which R₁ is as defined hereinbefore,
which compound of formula (VI) being treated with phenylmagnesium bromide under polar solvent to yield to the compound of formula (VII): 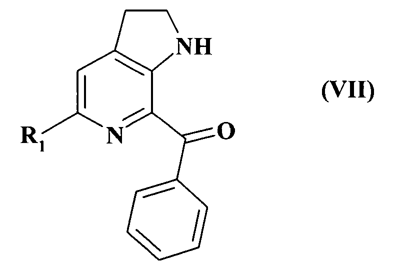 in which R₁ is as defined hereinbefore,
compound of formula (VII) being condensed with methyl glycinate hydrochloride in presence of pyridine, to yield to the compound of formula (VIII): 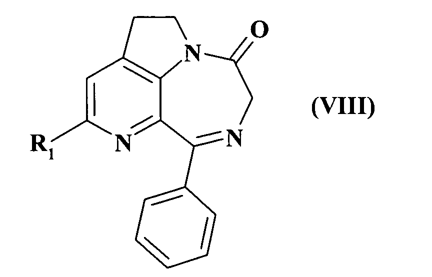 in which R₁ is as defined hereinbefore,
which compound of formula (VIII) is reacted with trisylazide to yield to the compound of formula (IX): 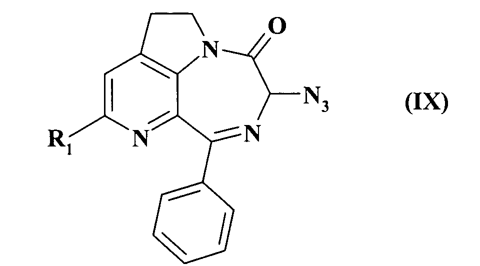 in which R₁ is as defined hereinbefore,
compound of formula (IX) being reduced under smooth conditions with triphenylphosphine in wet tetrahydrofurane to yield to the amino derivative of formula (X): 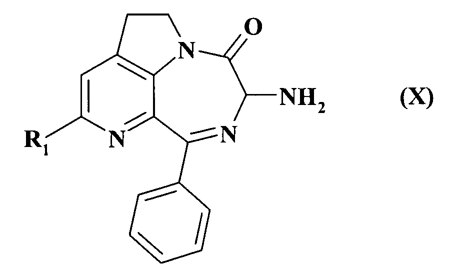 in which R₁ is as defined hereinbefore,
which compound of formula (X) being reacted under peptidic coupling conditions in basic medium using a classical coupling agent of organic synthesis with a compound of formula (XI): 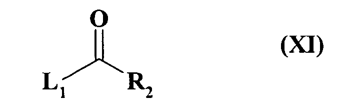 in which R₂ is as defined in the compounds of general formula (I) and L₁ represent a leaving group like halogen or (C₁-C₄)alkoxy,
to yield to the compound of formula (I), which are purified, where appropriate, according to a conventional purification technique, which are separated, where appropriate, into their different isomers according to a conventional separation technique, and which are converted, where appropriate, into addition salts thereof with a pharmaceutically-acceptable acid or base.

Compounds of formulae (II) and (XI) are either commercial or obtained easily by using classical reactions of organic synthesis well known by the man skilled in the art.

Compounds of formulae (IV), (V), (VI), (VII), (VIII) and (X) described in the hereinabove process constitute intermediate compounds for the synthesis of the final products of formula (I). These compounds of formula (IV), except 1-(2,3-dihydro-pyrrolo[2,3-*c*]pyridin-1-yl)acetate and *tert*-butyl 1-(2,3-dihydro-pyrrolo[2,3-*c*]pyridin-1-yl)carboxylate, and of formulae (V), (VI), (VII), (VIII) and (X) represent new compounds useful as intermediates.

The compounds of formula (I) are purified, where appropriate, according to a conventional purification technique, and separated, where appropriate, into their different isomers according to a conventional separation technique, and converted, where appropriate, into addition salts thereof with a pharmaceutically-acceptable acid or base.

Generally, isomers of the compounds of the invention are understood to be optical isomers such as enantiomers and diastereoisomers. More especially, pure enantiomeric forms of the compounds of the invention may be separated by starting from mixtures of enantiomers which are reacted with a racemate-separating agent that can be released, the said agent being itself in the form of a pure enantiomer, which allows the corresponding diastereoisomers to be obtained. The diastereoisomers are then separated according to the separation techniques well known to the person skilled in the art, such as crystallization or chromatography, and the separating agent is then removed using conventional techniques of organic synthesis, resulting in a pure enantiomer.

The compounds of the invention that are present in the form of a mixture of diastereoisomers are isolated in a pure form by using conventional separation techniques such as chromatography.

As mentioned above, compounds of formula (I) of the present invention are phosphodiesterase inhibitors, and more particularly inhibitors of the enzyme PDE4.

In this respect, their use is recommended for the treatment of diseases or complaints involving a therapy by PDE4 inhibition. By way of example, the use of the compounds of the present invention may be recommended for the treatment of cancer, acquired immunodeficiency syndrome, fibrosis, excessive scarring including excessive dermal scarring such as normal or abnormal dermal scarring following wounding or surgery, osteoarthritis, osteoporosis, multiple sclerosis, anxiety, depression, atopic dermatitis, rheumatoid arthritis, septic shock, immune diseases including disseminated lupus erythematous, psoriasis, graft rejection and allergic rhinitis, as well as diseases involving the production of TNFα and more particularly in the treatment of inflammatory complaints such as asthma, chronic obstructive bronchopneumopathy (COPD), post-ischaemic lesions, pulmonary hypertension, congestive cardiac insufficiency, acute respiratory distress syndrome, and chronic inflammatory diseases of the intestine (IBD) such as Crohn's disease and ulcerative colitis.

In a preferred embodiment, compounds of formula (I) of the present invention are useful for the preparation of a medicinal product intended for treating a disease selected from chronic obstructive bronchopneumopathy, asthma and chronic inflammatory diseases of the intestine such as Crohn's disease and ulcerative colitis.

More particularly, the compounds of formula (I) of the present invention are useful from treating a disease selected from chronic obstructive bronchopneumopathy and asthma.

The present invention also relates to pharmaceutical compositions comprising as active ingredient at least one compound of formula (I), an isomer thereof, a N-oxide thereof, or an addition salt thereof with a pharmaceutically-acceptable acid or base, alone or in combination with one or more pharmaceutically-acceptable, inert, non-toxic excipients or carriers.

Among the pharmaceutical compositions according to the invention, there may be mentioned more especially those that are suitable for oral, parenteral (intravenous, intramuscular or subcutaneous), per- or trans-cutaneous, intravaginal, rectal, nasal, perlingual, buccal, ocular or respiratory administration.

Pharmaceutical compositions according to the invention for parenteral injections especially include aqueous and non-aqueous sterile solutions, dispersions, suspension and emulsions, and also sterile powders for reconstituting injectable solutions or dispersions.

Pharmaceutical compositions according to the invention for oral administration in solid form especially include tablets or dragées, sublingual tablets, sachets, gelatin capsules and granules, for oral, nasal, buccal or ocular administration in liquid form, especially include emulsions, solutions, suspensions, drops, syrups and aerosols.

Pharmaceutical compositions for rectal or vaginal administration are preferably suppositories, and those for per- or trans-cutaneous administration especially include powders, aerosols, creams, ointments, gels and patches.

The pharmaceutical compositions mentioned hereinbefore illustrate the invention but do not limit it in any way.

Among the pharmaceutically acceptable, inert, non-toxic excipients or carriers there may be mentioned, by way of non-limiting example, diluents, solvents, preservatives, wetting agents, emulsifiers, dispersing agents, binders, swelling agents, disintegrating agents, retardants, lubricants, absorbents, suspending agents, colorants, aromatizing agents etc...

The useful dosage varies according to the age and weight of the patient, the administration route, the pharmaceutical composition used, the nature and severity of the disorder and the administration of any associated treatments. The dosage ranges from 2 mg to 1 g per day in one or more administrations. The compositions are prepared by methods that are common to those skilled in the art and generally comprise 0.5% to 60% by weight of active principle (compound of formula (I)) and 40% to 99.5% by weight of pharmaceutically acceptable excipients or carriers.

The examples that follow illustrate the invention but do not limit it in any way.

The starting materials used are products that are known or that are prepared according to known operating procedures. The various preparations yield synthetic intermediates that are useful in preparation of the compounds of the invention. Some of these intermediates are new compounds.

The structures of the compounds described in the Examples were determined according to the usual spectrophotometric techniques (infrared, nuclear magnetic resonance, mass spectrometry, ...)
In the experimental part:
- BOC₂O means di-*tert*-butyl dicarbonate
- TMEDA means *N,N,N',N'*-tetramethylethylenediamine
- LiHMDS means lithium hexamethyldisilazane
- DMF means dimethylformamide
- THF means tetrahydrofurane

### Example 1: N-(4-Methoxy-9-oxo-6-phenyl-1,2,8,9-tetrahydro-5,7,9a-triaza-benzo[cd]azulen-8-yl)-nicotinamide

### Step 1: tert-Butyl N-(6-methoxy-pyridin-3-yl)-carbamate

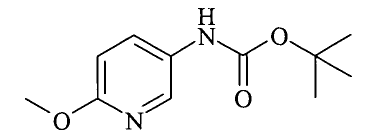

To a solution of 5-amino-2-methoxypyridine (25 g, 202 mmol) in 1,4-dioxane (300 ml) was added BOC₂O (48.4 g, 222 mmol) in one portion. The reaction mixture was heated at reflux for two hours, then concentrated in vacuum and further dissolved in dichloromethane. The organic phase was successively washed with HCl 1M, saturated NaHCO₃ and water, and dried over Na₂SO₄ and concentrated under reduced pressure. The crude product was purified by flash chromatography (dichloromethane/acetone: 95/5) afforded 35.4 g of the title compound as a salmon solid. Yield : 79%.
1H NMR (400 MHz, CDCl₃) δ 8.00 (d, 1H), 7.80 (br s, 1H) , 6.71 (d, 1H), 6.39 (br s, 1H), 3.90 (s, 3H), 1.50 (s, 9H).

### Step 2: tert-Butyl N (4-(2-hydroxy-ethyl)-6-methoxy-pyridin-3-yl)carbamate

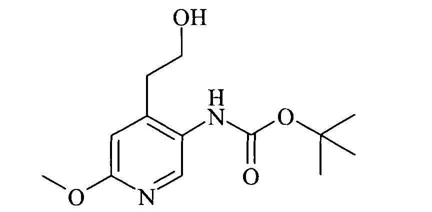

To a solution of the compound obtained in step 1 (17.5 g, 78 mmol) in dry diethyl ether (400 ml) under nitrogen atmosphere was added TMEDA (28.1 g, 242 mmol) in one portion. The reaction mixture was cooled to -78°C and *n*-butyl lithium (2.5 M solution in hexane, 100 ml, 234 mmol) was added maintaining a temperature less than -65°C. The reaction mixture was warmed to -10°C and held at this temperature for one hour (the mixture is milky), re-cooled to -78°C and ethylene oxide (8.4 g, 117 mmol) was added. The reaction mixture was allowed to warm slowly to 10°C and stirred until the solution turned to transparency. The reaction mixture was quenched with iced saturated NH₄Cl, diluted with ethyl acetate. The organic phase was separated and the aqueous phase extracted with ethyl acetate. The combined organic phases were dried over Na₂SO₄, concentrated *in vacuo* to yield the crude product as a brown oil. The crude material was purified by flash chromatography (cyclohexane/ethyl acetate 70/30 then 50/50) to yield the title compound as a yellow crystallizing oil (12.8 g, 61%).
¹H NMR (400 MHz, CDCl₃) δ8.14 (br s, 1H), 7.48 (br s, 1H), 6.54 (s, 1H), 3.85 (s, 3H), 3.82 (t, 2H, 5.55 Hz), 2.74 (t, 2H, 5.56H), 1.47 (s, 9H).

### Step 3: 2{5-[(tert-Butylcarbonyl)amino)-2-methoxypyridin-4-yl}ethylmethane sulphonate

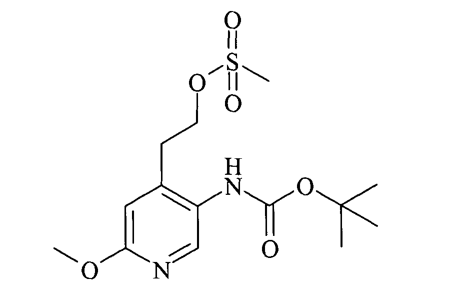

To a cooled solution of the compound obtained in step 2 (12.8 g, 48 mmol) in dry dichloromethane (200 ml) under nitrogen atmosphere was added at 0°C triethylamine (14.6 ml, 105 mmol) in one portion. To this mixture, methanesulphonyl chloride (6 g, 53 mmol) was added slowly keeping the temperature below 0°C. The reaction mixture was allowed to warm to ambient temperature and stirred 1h. The reaction mixture was quenched with saturated sodium bicarbonate (3 x 50 ml). The organic phase was separated and washed with water (20 ml). The combined organic phases were dried over Na₂SO₄ and concentrated *in vacuo* to yield the title compound as beige solid (15.6 g, 94 %).
¹H NMR (400 MHz, CDCl₃) δ 8.21 (s, 1H), 6.65 (s, 1H), 6.19 (br s, 1H), 4.46 (t, 2H), 3.9 (s, 3H), 3.01 (t, 2H), 2.97 (s, 3H), 1.49 (s, 9H).

### Step 4: tert-Butyl 5-methoxy-2,3-dihydro-pyrrolo[2,3-c]pyridine-1-carboxylate

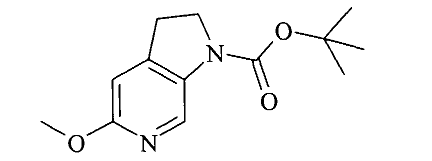

To a cooled solution of compound obtained in step 3 (15.6 g, 45 mmol) in tetrahydrofuran (220 ml) was added under nitrogen atmosphere at -78°C LiHMDS (1M solution in THF, 50 ml) maintaining a temperature less than -65°C. The reaction mixture was allowed to warm slowly to ambient temperature and stirred until complete. The reaction mixture was quenched in iced water and extracted with ether. The combined organic phases were dried over Na₂SO₄ and concentrated *in vacuo.* The resulting solid was triturated in ether to yield the title compound as a white solid (8.9 g, 80%).
¹H NMR (400 MHz, CDCl₃) δ 8.32 (2br s, 1H), 6.58 (s, 1H), 4.01 (t, 2H), 3.90 (s, 3H), 3.05 (t, 2H), 1.52 (s, 9H).

### Step 5: 7-Bromo-5-methoxy-2,3-dihydro-1H-pyrrolo[2,3-c]pyridine

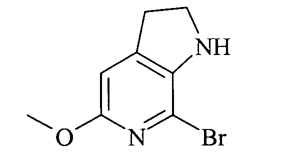

To a cooled solution of compound obtained in step 4 (11.7 g, 47 mmol) in hydrogen bromide 47% W/W (27 ml, 234 mmol) was added at 10°C *via* a dropping funnel a solution of hydrogen peroxide 50% W/W (3.5 ml, 61 mmol) diluted in 8 ml of water keeping the temperature less than 30°C. The reaction mixture was stirred at room temperature until complete by TLC. The reaction mixture was cooled to less than 10°C and basified to pH 7-8 with a solution of NaOH 1M. The organic phase was separated and the aqueous phase extracted with dichloromethane. The combined extracts were dried over Na₂SO₄, filtered and concentrated *in vacuo.* The crude material was purified by flash chromatography (dichloromethane/ethyl acetate: 99/01) to yield the title compound as a yellow oil (11.9 g 73%).
¹H NMR (400 MHz, CDCl₃) δ6.50 (s, 1H), 3.85 (s, 3H), 3.61 (t, 2H, 8.1 Hz), 3.10 (t, 2H, 8.1 Hz).

### Step 6: 5-Methoxy-2,3-dihydro-1H-pyrrolo[2,3-c]pyridine-7-carbonitrile

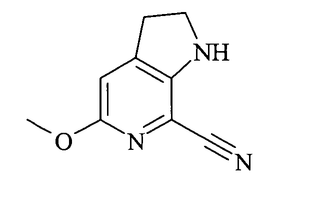

To a solution of compound obtained in step 5 (12 g, 52 mmol) in degassed DMF (100ml) under an atmosphere of nitrogen were added dppf (0.35 g, 0.64 mmol) and Pd(dba)₃ (0.500 g, 0.54 mmol) in one portion, followed by ZnCN₂ (3.66 g, 32 mmol). The reaction mixture was heated 3 hours to 130°C and stirred until complete by TLC. DMF was removed in *vacuum* and the residue was dissolved in ethyl acetate and ammoniac 14%. The organic phase was separated and the aqueous extracted with ethyl acetate. The combined organic fractions were dried over Na₂SO₄ and concentrated. The crude product was purified by column chromatography (dichloromethane/ethyl acetate: 98/02) to yield the title compound as a beige solid (6.4 g, 70%).
¹H NMR (400 MHz, CDCl₃) δ6.69 (s, 1H), 4.25 (br s, 1H), 3.88 (s, 3H), 3.72 (t, 2H), 3.10 (t, 2H).

### Step 7: 5-Methoxy-2,3-dihydro-1H pyrrolo-[2,3-c]pyridin-7-yl)-phenyl methanone

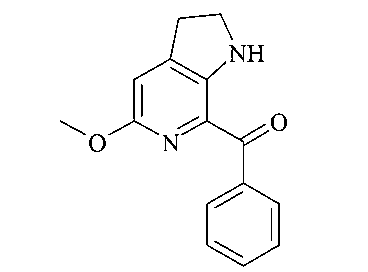

To a cooled solution of compound obtained in step 6 (0.5 g, 2.85 mmol) in THF (15 ml) under nitrogen atmosphere was added slowly at -5°C phenylmagnesium bromide (1M solution in THF, 15 ml) and stirred at this temperature for 15 minutes then warmed to room temperature until complete by TLC. The reaction mixture was quenched with a solution of ammonium chloride 1M and diluted with ethyl acetate. The reaction mixture was acidified to pH 1 using an aqueous solution of HCl 1M and the organic phase was separated. The aqueous phase was extracted with ethyl acetate. The combined organic fractions were dried over Na₂SO₄ and concentrated to dryness *in vacuum.* The crude material was purified by column chromatography (dichloromethane) to yield the title compound as a bright yellow solid (0.4 g, 55%).
¹H NMR (400 MHz, CDCl₃) δ 8.24 (d, 2H, 6.6 Hz), 7.42-7.51 (m, 3H), 7.06 (br s, 1H), 6.69 (s, 1H), 3.61 (m, 5H), 3.09 (t, 2H, 9.1 Hz).

### Step 8: 4-Methoxy-6-phenyl-1,2-dihydro-8H 5,7,9a-triaza-benzo[cd]azulen-9-one

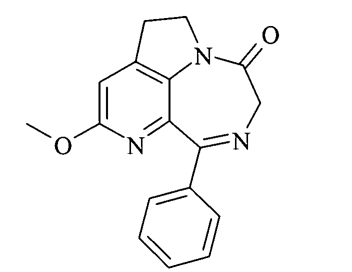

To a solution of compound obtained in step 7 (0.1 g, 0.40 mmol) in pyridine (25ml) was added methyl glycinate hydrochloride (0.054 g, 0.43 mmol). The mixture was heated two days at reflux (115°C) using a Dean Stark apparatus until complete by TLC. The reaction mixture was concentrated to dryness *in vacuo* and diluted in dichloromethane and washed with Na₂CO₃ 2.5%. The combined organic fractions were dried over Na₂SO₄ and concentrated to dryness *in vacuo.* The crude material was purified by column chromatography (dichloromethane/ethyl acetate: 80:20) to yield the title compound as a beige solid (0.050 g, 43%).
¹H NMR (400 MHz, CDCl₃) δ7.67 (d, 2H, 7.1 Hz), 7.35-7.42 (m, 3H), 6.82 (s, 1H), 4.44 (s, 2H), 4.27 (t, 2H, 7.6 Hz), 3.78 (s, 3H), 3.19 (t, 2H, 7.6 Hz).

### Step 9: 8-Azido-4-methoxy-6-phenyl-1,2-dihydro-8H-5,7,9a-triaza-benzo [cd] azulen-9-one

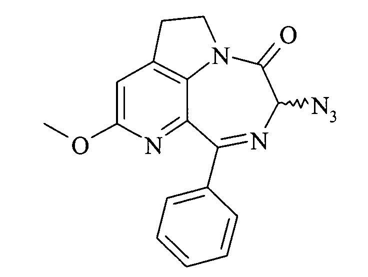

To a cooled suspension of potassium *tert*-butylate (0.042 g, 0.375 mmol) in THF (1 ml) was added at -78°C under nitrogen atmosphere, a solution of compound obtained in step 8 (0.1 g, 0.34 mmol) in THF (1 ml) dropwise. The reaction mixture turned dark blue. A solution of trisylazide (0.116 g, 0.375 mmol) in THF (1 ml) was added and the reaction mixture turned orange. The reaction mixture was stirred at -78°C for 5 minutes before glacial acetic acid was added (0.09 ml, 1.57 mmol) and allowed to warm to room temperature. The reaction mixture was diluted with dichloromethane and washed with a solution of NaHCO₃, water and brine. The combined organic fractions were dried over Na₂SO₄ and concentrated to dryness *in vacuo.* The crude material was purified by column chromatography (dichloromethane/ethyl acetate: 98:02) to yield the title compound as a solid (0.3 g, 26%).
¹H NMR (400 MHz, CDCl₃) δ 3.115 (m, 1H), 3.30 (m, 1H), 3.80 (s, 3H), 4.00 (m, 2H), 6.89 (s, 1H), 7.38-7.50 (m, 3H), 7.80 (d, 2H).

### Step 10: 8-Amino-4-methoxy-6-phenyl-1,2-dihydro-8H-5,7,9a-triaza-benzo[cd]azulen-9-one

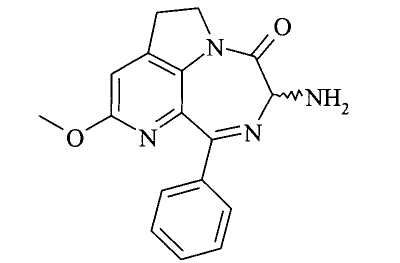

To a solution of compound obtained in step 9 (0.14 g, 0.42 mmol) in THF (3 ml) was added triphenylphosphine (0.22 g, 0.84) and water (0.33 ml). The reaction mixture was stirred at room temperature for 5 hours. The reaction mixture was concentrated to dryness *in vacuo.* The residue was diluted with ethyl acetate and extracted with a solution of HCl 1M and twice with water. The aqueous phase was basified to pH 12 with a solution of NaOH 1M and extracted with ethyl acetate. The combined organic phases were dried over Na₂SO₄ and concentrated to dryness *in vacuo* to yield the title compound as a solid (0.090 g, 69%), which was used in next step without any further purification.
'H NMR (400 MHz, CDCl₃) δ 2.50 (br s, 2H), 3.12 (m, 1H), 3.30 (m, 1H), 3.79 (s, 3H), 3.97 (m, 1H), 4.59 (s, 1H), 4.71 (m, 1H), 6.85 (s, 1H), 7.39-7.50 (m, 3H), 7.70 (d, 2H).

### Step 11: N-(4-Methoxy-9-oxo-6-phenyl-1,2,8,9-tetrahydro-5,7,9a-triaza-benzo[cd]azulen-8-yl)-nicotinamide

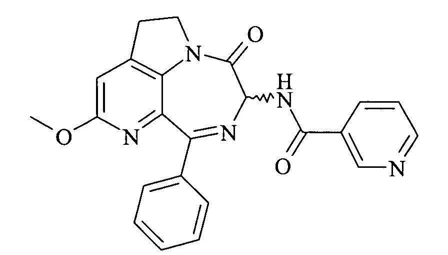

To a solution of compound obtained in step 10 (0.070 g, 0.23 mmol), in dichloromethane (3 ml) was added nicotinic acid (0.030 g, 0.24 mmol) and *O*-[(ethoxycarbonyl)cyanomethyleneamino]-*N,N,N',N'*-tetramethyluronium tetrafluoroborate (TOTU) (0.075 g, 0.23 mmol) in one portion. The reaction mixture was cooled to 0-5°C and diisopropylethylamine (0.060 g, 0.46 mmol) was added dropwise. The yellow solution was stirred overnight at room temperature. The reaction mixture was diluted with dichloromethane and washed with water, a solution of NaHCO₃ and brine. The organic phase was dried over Na₂SO₄ and concentrated to dryness *in vacuo.* The crude material was purified by column chromatography (dichloromethane/methanol 100/0, then 98/02 and 97/03) to yield to the desired product, which was triturated in a mixture of ether and pentane (0.080 g, 84%). Both enantiomers were separated by chiral preparative HPLC (column Chiralcel@ OD-H 20x250 mm, 5µm, 17 ml/min, acetonitrile 100%). Rt = 5.79 min and 8.15 min.
¹H NMR (500 MHz, DMSO) δ 3.17 (m, 1H), 3.37 (m, 1H), 3.75 (s, 3H), 3.96 (m, 1H), 4.47 (m, 1H), 5.60 (d, 1H), 7.15 (s, 1H), 7.43 (m, 2H), 7.49 (m, 1H), 7.56 (m, 1H), 7.68 (d, 2H), 8.39 (m, 1H), 8.76 (d, 1H), 9.18 (s, 1H), 9.96 (d, 1H).

### Evaluation of the in vitro activity of the compounds of the examples

The capacity of the compounds of formula (I) of the invention to inhibit cyclic nucleotide phosphodiesterases is evaluated by measuring their IC₅₀ (concentration needed to inhibit 50% of the enzymatic activity). In the case of PDE4 enzymes, this value is compared to the IC₅₀ value for rolipram, a reference inhibitor for PDE4 enzymes.

The type 4 phosphodiesterases are obtained from a cytosolic preparation extracted from a cell line of human origin, U937, according to the method adapted from T. J. Torphy et al., 1992, J. Pharm. Exp. Ther. 263: 1195-1205.

The other types of phosphodiesterases are obtained during a partial purification by FPLC on a Mono Q column (anion exchange column) according to a method adapted from Lavan B. E., Lakey T., Houslay M. D. Biochemical Pharmacology, 1989, 38(22), 4123-4136, and Silver P.J et al., 1988, Eur. J. Pharmacol. 150: 85-94, either starting with cell lines of human origin for PDE1 (monocyte line TPH1) and PDE5 (line derived from an adenocarcinoma MCF7), or starting with dog aorta for PDE3, or, for human PDE3A, starting with a cloning of genes in SF21 insect cells into baculovirus, according to the method adapted from Luckow, V. A. et al., 1991 in Recombinant DNA Technology & Applications., eds. Prokop, Bajpai, R. K. & Ho, C.S., pp 97-152.

The measurement of the enzymatic activity for the various types of PDE, and in particular the PDE4 enzymes, is carried out according to a method adapted from W. J. Thomson et al. 1979, Advances in Cyclic Nucleotide Research, Vol. 10: 69-92, ed. G. Brooker et al. Raven Press, NY.

For the determination of the IC₅₀ value, the enzymatic activity is measured in the presence of the inhibitor in a concentration range from 0.1 to 100 µM.

Using this test, the racemate of compound of Example 1, the first enantiomer (Rt=5.79 min) of compound of Example 1, and the second enantiomer (Rt = 8.15 min) of compound of Example 1 show respectively an IC₅₀ at 6.1 µM, 4.3 µM, and 73 µM.

## Claims

1. Compounds of formula (I): 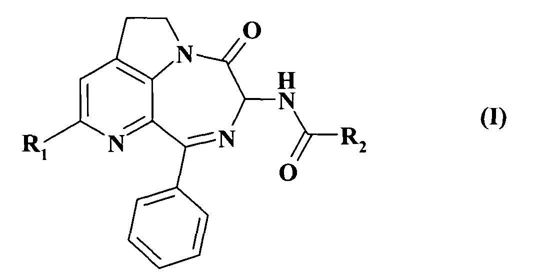 **characterized in that**:
• R₁ represents a group selected from hydrogen atom, methyl, methoxy, hydroxy, amino, dimethylamino, acetamido, pyrrolidin-1-yl, and hydroxymethyl;
• R₂ represent a group selected from phenyl, pyridyl, pyrimidyl, quinolyl, isoquinolyl, indolyl, pyrolyl, [1,2,3]-triazolyl, benzo[c]isoxazolyl, thienyl, pyrazolyl, isothiazolyl, imidazolyl, benzofuranyl, pyrazolo[5,1-c][1,2,4]triazyl each of these groups being optionally substituted from 1 to 3 groups, identical or different independently of each other, selected from halogen, trifluoromethyl, (C₁-C₄)alkyl, (C₁-C₄)alkoxy, hydroxy, amino, acetamido, tert-butyloxycarbonylamino, cycloalkylcarbonylamino, sulfonamide, nitro, acetylmethoxy, cyclopentyloxy;
and optionally, their optical isomers, and addition salts thereof with a pharmaceutically acceptable acid or base.

2. Compounds of formula (I) according to claim 1 **characterized in that**:
• R₁ represents a group selected from methyl, methoxy, amino and acetamido;
• R₂ represents a group selected from phenyl, pyridin-3-yl, and pyridin-4-yl, each of these groups being optionally substituted from 1 to 3 groups, identical or different independently of each other selected from halogen, methoxy and amino;
and optionally, their optical isomers, and addition salts thereof with a pharmaceutically acceptable acid or base.

3. Compounds of formula (I) according to anyone of claims 1 or 2 **characterized in that** R₂ represents a pyridin-4-yl or a pyridin-3-yl group optionally substituted from 1 to 3 groups as defined in claim 1, and optionally, their optical isomers, and addition salts thereof with a pharmaceutically acceptable acid or base.

4. Compounds of formula (I) according to anyone of claims 1 or 2 **characterized in that** R₁ represents a group selected from methoxy and amino, and R₂ represents a pyridin-3-yl, and optionally, their optical isomers, and addition salts thereof with a pharmaceutically acceptable acid or base.

5. A compound of formula (I) according to claim 1, which is:
• *N*-(4-methoxy-9-oxo-6-phenyl-1,2,8,9-tetrahydro-5,7,9a-triaza-benzo[*cd*]azulen-8-yl)-nicotinamide,
its optical isomers, and addition salt thereof with a pharmaceutically acceptable acid or base.

6. A compound of formula (I) according to claim 1, which is the (1R) *N*-(4-methoxy-9-oxo-6-phenyl-1,2,8,9-tetrahydro-5,7,9a-triaza-benzo[*cd*]azulen-8-yl)-nicotinamide, and addition salt thereof with a pharmaceutically acceptable acid or base.

7. A compound of formula (I) according to claim 1, which is the (1S) *N*-(4-methoxy-9-oxo-6-phenyl-1,2,8,9-tetrahydro-5,7,9a-triaza-benzo[*cd*]azulen-8-yl)-nicotinamide, and addition salt thereof with a pharmaceutically acceptable acid or base.

8. A process for the preparation of the compound of formula (I) **characterized in that** there is used as starting material a compound of formula (II): 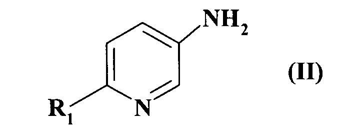 in which R₁ is as defined in the compound of general formula (I),
compound of formula (II) in which the primary amino group is protected in a first step by a usual protective group (G₁) used classically in organic synthesis, then is treated with oxirane or bromoethanol, in presence of a strong base to yield the compound of formula (III): 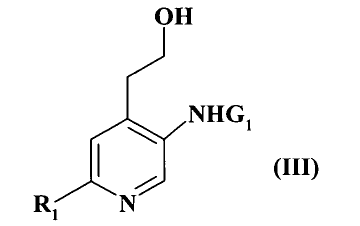 in which R₁ and G₁ are as defined hereinbefore,
compound of formula (III) which is reacted with methanesulfonyl chloride to yield the corresponding mesylate intermediate (in the place of the primary alcohol), which is treated directly in polar condition with LiHMDS to yield to the cyclized compound of formula (IV): 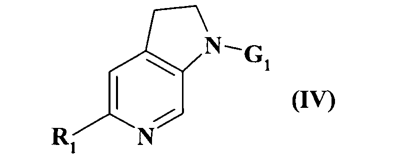 in which R₁ and G₁ are as defined hereinbefore,
compound of formula (IV) which is treated with hydrogen bromide in the presence of hydrogen peroxide to yield to the corresponding 7-bromo-pyrrolo[2,3-c]pyridin of formula (V): 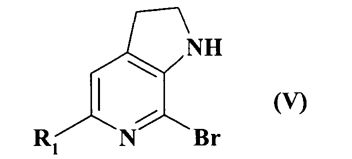 in which R₁ is as defined hereinbefore,
compound of formula (V), which is reacted with zinc cyanide under palladium condition or with cupper cyanide under heating or micro-wave conditions to yield to the corresponding cyanide compound of formula (VI): 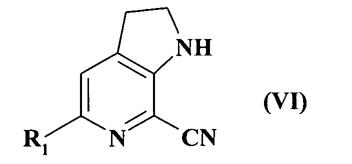 in which R₁ is as defined hereinbefore,
which compound of formula (VI) being treated with phenylmagnesium bromide under polar solvent to yield to the compound of formula (VII): 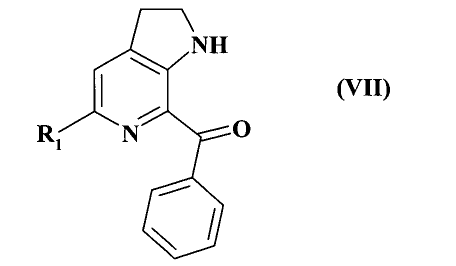 in which R₁ is as defined hereinbefore,
compound of formula (VII) being condensed with methyl glycinate hydrochloride in presence of pyridine, to yield to the compound of formula (VIII): 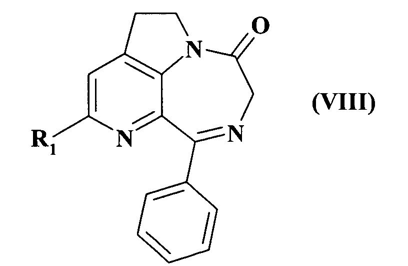 in which R₁ is as defined hereinbefore,
which compound of formula (VIII) is reacted with trisylazide to yield to the compound of formula (IX): 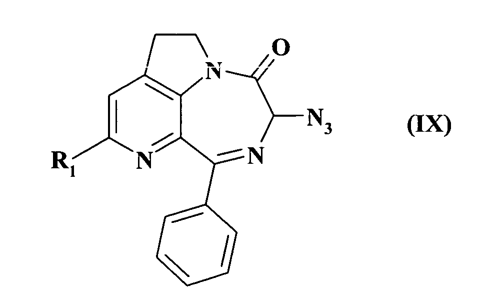 in which R₁ is as defined hereinbefore,
compound of formula (IX) being reduced under smooth conditions with triphenylphosphine in wet tetrahydrofurane to yield to the amino derivative of formula (X): 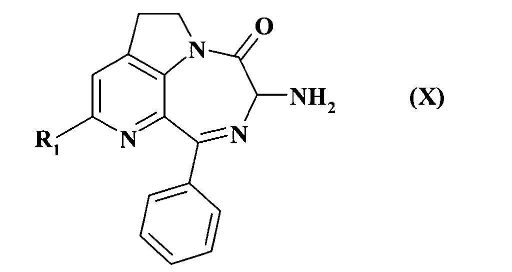 in which R₁ is as defined hereinbefore,
which compound of formula (X) being reacted under peptidic coupling conditions in basic medium using a classical coupling agent of organic synthesis with a compound of formula (XI): 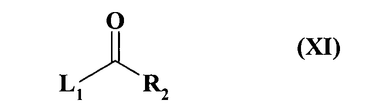 in which R₂ is as defined in the compounds of general formula (I) and L₁ represent a leaving group like halogen or (C₁-C₄)alkoxy,
to yield to the compound of formula (I), which are purified, where appropriate, according to a conventional purification technique, which are separated, where appropriate, into their different isomers according to a conventional separation technique, and which are converted, where appropriate, into addition salts thereof with a pharmaceutically-acceptable acid or base.

9. Intermediate compounds of formula (IV): 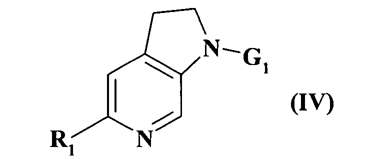 in which R₁ is as defined in the compound of formula (I) in claim 1, and G₁ represents a protecting group of the amino group classically used in organic synthesis, except that (IV) does not represent 1-(2,3-dihydro-pyrrolo[2,3-c]pyridin-1-yl)acetate and *tert-*butyl 1-(2,3-dihydro-pyrrolo[2,3-*c*]pyridin-1-yl)carboxylate.

10. Intermediate compounds of formula (V): 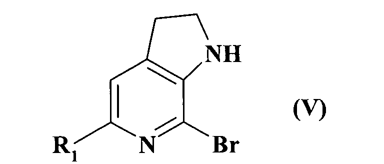 in which R₁ is as defined in the compound of formula (I) in claim 1.

11. Intermediate compounds of formula (VI): 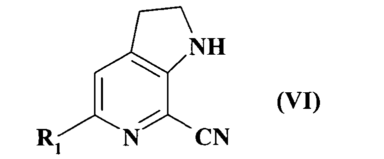 in which R₁ is as defined in the compound of formula (I) in claim 1.

12. Intermediate compounds of formula (VII): 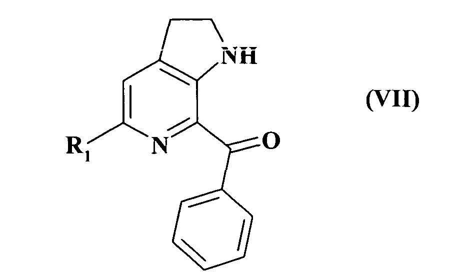 in which R₁ is as defined in the compound of formula (I) in claim 1.

13. Intermediate compounds of formula (VIII): 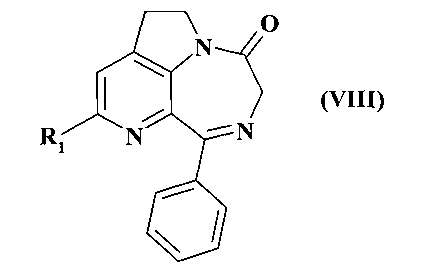 in which R₁ is as defined in the compound of formula (I) in claim 1.

14. Intermediate compounds of formula (X): 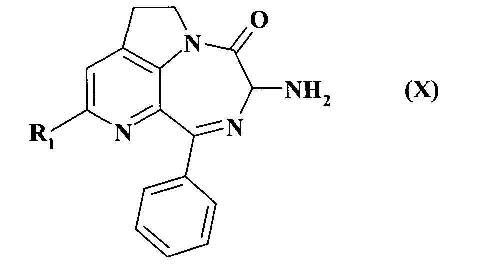 in which R₁ is as defined in the compound of formula (I) in claim 1.

15. A method for treating a living body afflicted with a disease where the inhibition of phosphodiesterase type 4 is involved, comprising the step of administering to the living body an amount of a compound of claim 1 which is effective for alleviation of said conditions.

16. A method for treating a living body afflicted with a disease selected from cancer, acquired immunodeficiency syndrome, fibrosis, excessive scarring including excessive dermal scarring such as normal or abnormal dermal scarring following wounding or surgery, osteoarthritis, osteoporosis, multiple sclerosis, anxiety, depression, atopic dermatitis, rheumatoid arthritis, septic shock, immune diseases including disseminated lupus erythematous, psoriasis, graft rejection and allergic rhinitis, as well as diseases involving the production of TNFα, inflammatory complaints such as asthma, chronic obstructive bronchopneumopathy (COPD), post-ischaemic lesions, pulmonary hypertension, congestive cardiac insufficiency, acute respiratory distress syndrome, and chronic inflammatory diseases of the intestine (IBD) such as Crohn's disease and ulcerative colitis, comprising the step of administering to the living body an amount of a compound of claim 1 which is effective for alleviation of said conditions.

17. A pharmaceutical composition comprising as active ingredient an effective amount of a compound as claimed in claim 1, alone or in combination with one or more pharmaceutically-acceptable excipients or carriers.

18. A pharmaceutical composition useful in the method of Claim 15 comprising as active ingredient an effective amount of a compound as claimed in claim 1, together with one or more pharmaceutically-acceptable excipients or carriers.

19. Use of a compound according to Claim 1, for the preparation of a medicinal product intended for treating a disease involving therapy by inhibition of type 4 phosphodiesterase.

20. Use according to Claim 19, **characterized in that** the disease is cancer, acquired immunodeficiency syndrome, fibrosis, excessive scarring including excessive dermal scarring such as normal or abnormal dermal scarring following wounding or surgery, osteoarthritis, osteoporosis, multiple sclerosis, anxiety, depression, atopic dermatitis, rheumatoid arthritis, septic shock, immune diseases including disseminated lupus erythematous, psoriasis, graft rejection and allergic rhinitis, as well as diseases involving the production of TNFα, inflammatory complaints such as asthma, chronic obstructive bronchopneumopathy (COPD), post-ischaemic lesions, pulmonary hypertension, congestive cardiac insufficiency, acute respiratory distress syndrome, and chronic inflammatory diseases of the intestine (IBD) such as Crohn's disease and ulcerative colitis.

21. Use according to Claim 19, **characterized in that** the disease is selected from chronic obstructive bronchopneumopathy, asthma and chronic inflammatory diseases of the intestine (IBD) such as Crohn's disease and ulcerative colitis.

22. Use according to Claim 19, **characterized in that** the disease is selected from chronic obstructive bronchopneumopathy and asthma.
